(19) Europäisches Patentamt
European Patent Office
Office européen des brevets

(11) EP 0 945 442 A1

(12) **EUROPEAN PATENT APPLICATION**

(43) Date of publication:
**29.09.1999 Bulletin 1999/39**

(51) Int. Cl.[6]: **C07D 239/50**, C07D 401/12, A61K 31/505

(21) Application number: **98201587.7**

(22) Date of filing: **14.05.1998**

(84) Designated Contracting States:
**AT BE CH CY DE DK ES FI FR GB GR IE IT LI LU MC NL PT SE**
Designated Extension States:
**AL LT LV MK RO SI**

(30) Priority: **27.03.1998 US 79632 P**

(71) Applicant:
**JANSSEN PHARMACEUTICA N.V.**
**2340 Beerse (BE)**

(72) Inventors:
- **Kukla, Michael Joseph**
  **Maple Glen, Pennsylvania 19002 (US)**
- **Ludovici, Donald W.**
  **Quakertown, Pennsylvania 18951 (US)**
- **Ho, Chih Yung**
  **Lansdale, Pennsylvania 19446 (US)**
- **De Corte, Bart**
  **Southampton, Pennsylvania 18966 (US)**
- **Janssen, Marcel**
  **2350 Vosselaar (BE)**
- **Janssen, Paul Adriaan Jan**
  **2350 Vosselaar (BE)**

(54) **Trisubstituted pyrimidine derivatives**

(57) This invention concerns the use of the compounds of formula

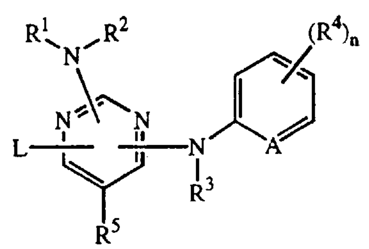

(I)

the *N*-oxides, the pharmaceutically acceptable addition salts and the stereochemically isomeric forms thereof, wherein A is CH or N; n is 0, 1, 2, 3 or 4; and in case A is CH, then n may also be 5; $R^1$ and $R^2$ are each independently selected from hydrogen, hydroxy, $C_{1-12}$alkyl, $C_{1-12}$alkyloxy, $C_{1-12}$alkylcarbonyl, $C_{1-12}$alkyloxycarbonyl, aryl, amino, mono- or di($C_{1-12}$alkyl)amino, mono- or di($C_{1-12}$alkyl)aminocarbonyl wherein each of the aforementioned $C_{1-12}$alkyl groups may optionally and each individually be substituted; or $R^1$ and $R^2$ taken together may form pyrrolidinyl, piperidinyl, morpholinyl, azido or mono- or di($C_{1-12}$alkyl)amino$C_{1-4}$alkylidene; $R^3$ is hydrogen, aryl, $C_{1-6}$alkylcarbonyl, optionally substituted $C_{1-6}$alkyl, $C_{1-6}$alkyloxycarbonyl; and each $R^4$ independently is hydroxy, halo, $C_{1-6}$alkyl, $C_{1-6}$alkyloxy, cyano, aminocarbonyl, nitro, amino, trihalomethyl or trihalomethyloxy; $R^5$ is hydrogen or $C_{1-4}$alkyl; L is optionally substituted $C_{1-10}$alkyl, $C_{3-10}$alkenyl, $C_{3-10}$alkynyl, $C_{3-7}$cycloalkyl; or L is -X-$R^6$ wherein $R^6$ is optionally substituted phenyl; and X is -$NR^3$-, -NH-NH-, -N=N-, -O-, -S-, -S(=O)- or -$S(=O)_2$-; aryl is optionally substituted phenyl; Het is an optionally substituted aliphatic or optionally substituted aromatic heterocyclic radical for the manufacture of a medicine for the treatment of subjects suffering from HIV (Human Immunodeficiency Virus) infection. It further relates to new compounds being a subgroup of the compounds of formula (I), their preparation and compositions comprising them.

EP 0 945 442 A1

## Description

The present invention is concerned with trisubstituted pyrimidine derivatives having HIV replication inhibiting properties. The invention further relates to methods for their preparation and pharmaceutical compositions comprising them. The invention also relates to the use of said compounds in the manufacture of a medicant useful for the treatment of subjects suffering from HIV (Human Immunodeficiency Virus) infection.

Compounds structurally related to the present compounds are disclosed in the prior art

JP-2,052,360, JP-2,308,248, JP-9,080,676 and JP-9,068,784 disclose a number of trisubstituted pyrimidines useful in photographic material. JP-8,199,163 discloses trisubstituted pyrimidines useful in an organic electroluminescent device. JP-2,300,264 and GB-1,477,349 disclose pyrimidinetriamines for their use in the dye industry.

J. Indian Chem. Soc. (1975), 52(8), 774-775 discloses the synthesis of some bis(arylamino)pyrimdines. J. Heterocycl. Chem. (1973), 10(2), 167-171 discloses the condensation of various aminopyrimidines with picryl halides. J. Org. Chem. (1961), 26, 4433-4440 discloses several triaminopyrimidines as intermediates in the synthesis of triazolo[4,5-d]pyrimidines.

WO 91/18887 discloses diaminopyrimidines as gastric acid secretion inhibitors.

Unexpectedly, it has now been found that the compounds of formula (I) effectively inhibit the replication of the Human Immunodeficiency Virus (HIV) and consequently may be useful for the treatment of individuals infected by HIV.

The present invention concerns the use of the compounds of formula

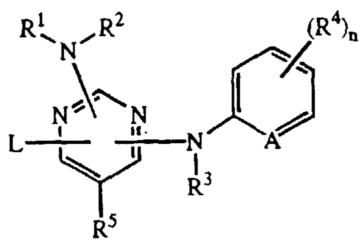

(I)

the *N*-oxides, the pharmaceutically acceptable addition salts and the stereochemically isomeric forms thereof, wherein

A is CH or N;

n is 0, 1, 2, 3 or 4; and in case A is CH, then n may also be 5;

$R^1$ and $R^2$ are each independently selected from hydrogen, hydroxy, $C_{1-12}$alkyl, $C_{1-12}$alkyloxy, $C_{1-12}$alkylcarbonyl, $C_{1-12}$alkyloxycarbonyl, aryl, amino, mono- or di($C_{1-12}$alkyl)amino, mono- or di($C_{1-12}$alkyl)aminocarbonyl wherein each of the aforementioned $C_{1-12}$alkyl groups may optionally and each individually be substituted with one or two substituents each independently selected from hydroxy, $C_{1-6}$alkyloxy, hydroxy$C_{1-6}$alkyloxy, carboxyl, $C_{1-6}$alkyloxycarbonyl, cyano, amino, imino, aminocarbonyl, aminocarbonylamino, mono- or di($C_{1-6}$alkyl)amino, aryl and Het; or

$R^1$ and $R^2$ taken together may form pyrrolidinyl, piperidinyl, morpholinyl, azido or mono- or di($C_{1-12}$alkyl)amino$C_{1-4}$alkylidene;

$R^3$ is hydrogen, aryl, $C_{1-6}$alkylcarbonyl, $C_{1-6}$alkyl, $C_{1-6}$alkyloxycarbonyl, $C_{1-6}$alkyl substituted with $C_{1-6}$alkyloxycarbonyl; and

each $R^4$ independently is hydroxy, halo, $C_{1-6}$alkyl, $C_{1-6}$alkyloxy, cyano, aminocarbonyl, nitro, amino, trihalomethyl or trihalomethyloxy ;

$R^5$ is hydrogen or $C_{1-4}$alkyl;

L is $C_{1-10}$alkyl, $C_{3-10}$alkenyl, $C_{3-10}$alkynyl, $C_{3-7}$cycloalkyl, or $C_{1-10}$alkyl substituted with one or two substituents independently selected from

- $C_{3-7}$cycloalkyl,
- indolyl or indolyl substituted with one, two, three or four substituents each independently selected from halo, $C_{1-6}$alkyl, $C_{1-6}$alkyloxy, cyano, aminocarbonyl, nitro, amino, trihalomethyl, trihalomethyloxy and $C_{1-6}$alkylcarbonyl,
- phenyl or phenyl substituted with one, two, three, four or five substituents each independently selected from halo, hydroxy, $C_{1-6}$alkyl, $C_{1-6}$alkyloxy, cyano, aminocarbonyl, nitro, amino, trihalomethyl, trihalomethyloxy and $C_{1-6}$alkylcarbonyl; or

L is -X-$R^6$ wherein

$R^6$ is phenyl or phenyl substituted with one, two, three, four or five substituents each independently selected from halo, $C_{1-6}$alkyl, $C_{1-6}$alkyloxy, $C_{1-6}$alkylcarbonyl, cyano, nitro and trifluoromethyl; and
X is $-NR^3-$, -NH-NH-, -N=N-, -O-, -S-, -S(=O)- or $-S(=O)_2-$;

aryl is phenyl or phenyl substituted with one, two, three, four or five substituents each independently selected from halo, $C_{1-6}$alkyl, $C_{1-6}$alkyloxy, cyano, nitro and trifluoromethyl;
Het is an aliphatic or aromatic heterocyclic radical; said aliphatic heterocyctic radical is selected from pyrrolidinyl, piperidinyl, homopiperidinyl, piperazinyl, morpholinyl, tetrahydrofuranyl and tetrahydrothienyl wherein each of said aliphatic heterocyclic radical may optionally be substituted with an oxo group; and said aromatic heterocyclic radical is selected from pyrrolyl, furanyl, thienyl, pyridinyl, pyrimidinyl, pyrazinyl and pyridazinyl wherein each of said aromatic heterocyclic radical may optionally be substituted with hydroxy;

for the manufacture of a medicine for the treatment of subjects suffering from HIV (Human Immunodeficiency Virus) infection.

The present invention also relates to a method of treating warm-blooded animals suffering from HIV (Human Immunodeficiency Virus) infection. Said method comprises the administration of a therapeutically effective amount of a compound of formula (I) or a *N*-oxide form, a pharmaceutically acceptable addition salt or a stereochemically isomeric form thereof in admixture with a pharmaceutical carrier.

This invention also concerns novel compounds of formula

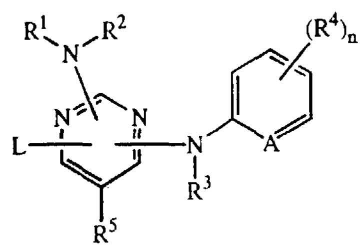

(I')

wherein $R^1$ to $R^5$, n and A are as defined under formula (I)
L is $C_{1-10}$alkyl substituted with one or two substituents independently selected from phenyl or phenyl substituted with one, two, three, four or five substituents each independently selected from halo, hydroxy, $C_{1-6}$alkyl, $C_{1-6}$alkyloxy, cyano, aminocarbonyl, nitro, amino, trihalomethyl, trihalomethyloxy and $C_{1-6}$alkylcarbonyl; or
L is $-X-R^6$ wherein

$R^6$ is phenyl or phenyl substituted with one, two, three, four or five substituents each independently selected from halo, $C_{1-6}$alkyl, $C_{1-6}$alkyloxy, $C_{1-6}$alkylcarbonyl, cyano, nitro and trifluoromethyl; and

X is $-NR^3-$, -NH-NH-, -N=N-, -O-, -S-, -S(=O)- or $-S(=O)_2-$;
with the proviso that compounds

(a) *N*2-hydroxy-*N*2-methyl-*N*4,*N*6-diphenyl-2,4,6-pyrimidinetriamine;
(b) *N*,*N*,*N'*,*N'*,*N''*,*N''*-hexakis(3-methylphenyl)-2,4,6-pyrimidinetriamine;
(c) *N*4-methyl-*N*2-(2-methylphenyl)-*N*4-phenyl-2,4,6-pyrimidinetriamine;
(d) *N*4-methyl-*N*2-(2-methylphenyl)-*N*4-phenyl-6-(phenylmethyl)-2,4-pyrimidinediamine;
(e) *N*4-(2-methylphenyl)-6-(phenylmethyl)-2,4-pyrimidinediamine;
(f) *N*,*N'*,*N''*-tris(4-methoxyphenyl)-2,4,6-pyrimidinetriamine;
(g) *N*,*N'*-bis(4-hexylphenyl)-6-(4-methoxyphenoxy)-2,4-pyrimidinediamine;
(h) *N*2,*N*4-bis(4-hexylphenyl)-*N*6,*N*6-dimethyl-2,4,6-pyrimidinetriamine;
(i) *N*,*N'*,*N''*-tris(4-hexylphenyl)-2,4,6-pyrimidinetriamine;
(j) *N*2,*N*2-dimethyl-*N*4,*N*6-bis(4-methylphenyl)-2,4,6-pyrimidinetriamine;
(k) *N*,*N'*,*N''*-tris(4-methylphenyl)-2,4,6-pyrimidinethamine;
(l) *N*,*N'*,*N''*-triphenyl-2,4,6-pyrimidinetriamine;
(m) *N*,*N*,*N'*,*N'*,*N''*,*N''*-hexakis(4-ethoxyphenyl)-2,4,6-pyrimidinetriamine;
(n) *N*4,*N*6-bis(2-chlorophenyl)-2,4,6-pyrimidinetriamine;

(o) *N*4,*N*6-bis(3-chlorophenyl)-2,4,6-pyrimidinetriamine;
(p) *N*4,*N*6-bis(2-ethoxyphenyl)-2,4,6-pyrimidinetriamine;
(q) *N*4,*N*6-bis(4-ethoxyphenyl)-2,4,6-pyrimidinetriamine;
(r) *N*4,*N*6-bis(2-methylphenyl)-2,4,6-pyrimidinetriamine;
(s) *N*4,*N*6-bis(4-bromophenyl)-2,4,6-pyrimidinetriamine;
(t) *N*4,*N*6-bis(4-methylphenyl)-2,4,6-pyrimidinetriamine;
(u) *N*2,*N*4-bis(4-methoxyphenyl)-2,4,6-pyrimidinetriamine;
(v) *N*2,*N*4-bis(4-methylphenyl)-2,4,6-pyrimidinethamine;
(w) *N*,*N'*,*N''*-tris(2,4,6-trinitrophenyl)-2,4,6-pyrimidinetriamine;
(x) *N*4,*N*6-bis(4-chlorophenyl)-2,4,6-pyrimdinetriamine;
(y) *N*4,*N*6-bis(4-methoxyphenyl)-2,4,6-pyrimidinetriamine;
(z) *N*2,*N*4,*N*6-trimethyl-*N*2,*N*4,*N*6-triphenyl pyrimidine-2,4,6-triyltriamine;
(aa) *N*4,*N*4-dimethyl-*N*2,*N*6-di-*p*-tolyl-pyrimidine-2,4,6-triyltriamine; and
(bb) *N*2,*N*4-diphenyl-pyrimidine-2,4,6-triyltriamine;

are not included; the *N*-oxides, the pharmaceutically acceptable addition salts and the stereochemically isomeric forms thereof.

The proviso is intended to exclude compound (a) disclosed in JP-9,080,676 and JP-9,068,784; compound (b) disclosed in JP-8,199,163; compounds (c) to (e) disclosed in WO 91/18887; compounds (f) to (i) disclosed in JP-2,308,248; compounds (j) to (l) disclosed in JP-2,300,264; compound (m) disclosed in JP-2,052,360; compounds (n) to (v) disclosed in Sen D. And Dutta P. J. Indian Chem. Soc. (1975), 52(8), 774-775; compound (w) disclosed in Harris et al. J. Heterocycl. Chem. (1973), 10(2), 167-171; compounds (x) and (y) disclosed in Fulmer-Shealy et al. J. Org. Chem. (1961), 26, 4433-4440; and compounds (z) to (bb) disclosed in Beilstein.

As used in the foregoing definitions and hereinafter halo defines fluoro, chloro, bromo and iodo; $C_{1-4}$alkyl encompasses the straight and branched chained saturated hydrocarbon radicals having from 1 to 4 carbon atoms such as, for example, methyl, ethyl, propyl, butyl and the like; $C_{1-6}$alkyl encompasses the straight and branched chained saturated hydrocarbon radicals as defined in $C_{1-4}$alkyl as well as the higher homologues thereof containing 5 or 6 carbon atoms such as, for example pentyl or hexyl; $C_{1-10}$alkyl encompasses the straight and branched chained saturated hydrocarbon radicals as defined in $C_{1-6}$alkyl as well as the higher homologues thereof containing 7 to 10 carbon atoms such as, for example, heptyl, octyl, nonyl or decyl; $C_{1-12}$alkyl encompasses the straight and branched chained saturated hydrocarbon radicals as defined in $C_{1-10}$alkyl as well as the higher homologues thereof containing 11 or 12 carbon atoms such as, for example, undecyl, dodecyl and the like; $C_{1-4}$alkylidene defines bivalent straight and branched chained hydrocarbons having from 1 to 4 carbon atoms such as, for example, methylene, ethylidene, propylidene, butylidene and the like; $C_{3-7}$cycloalkyl is generic to cyclopropyl, cyclobutyl, cyclopentyl, cyclohexyl and cycloheptyl; $C_{3-10}$alkenyl defines straight and branch chained hydrocarbon radicals containing one double bond and having from 3 to 10 carbon atoms such as, for example, 2-propenyl, 2-butenyl, 2-pentenyl, 3-pentenyl, 3-methyl-2-butenyl, 3-hexenyl, 3-heptenyl, 2-octenyl, 2-nonenyl, 2-decenyl and the like, whereby the carbon atom attached to the pyrimidine ring is preferably an aliphatic carbon atom; $C_{3-10}$alkynyl defines straight and branch chained hydrocarbon radicals containing one triple bond and having from 3 to 10 carbon atoms such as, for example, 2-propynyl, 2-butynyl, 2-pentynyl, 3-pentynyl, 3-methyl-2-butynyl, 3-hexynyl, 3-heptynyl, 2-octynyl, 2-nonynyl, 2-decynyl and the like, whereby the carbon atom attached to the triazine ring is preferably an aliphatic carbon atom.

It is to be understood that the three substituents [L, $NR^1R^2$ and $NR^3$(substituted phenyl or pyridinyl)] on the pyrimidine ring can be on any free position of the pyrimidine ring. Thus, given the following numbering of the pyrimidine ring

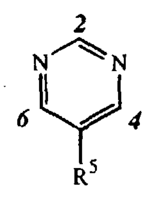

the three substituents may be connected to the pyrimidine ring in three different ways :

2-L, 4-$NR^1R^2$, 6-$NR^3$(substituted phenyl or pyridinyl); or
4-L, 2-$NR^1R^2$, 6-$NR^3$(substituted phenyl or pyridinyl); or
6-L, 4-$NR^1R^2$, 2-$NR^3$(substituted phenyl or pyridinyl).

The positions 4 and 6 are equivalent to one another. For instance, substitution pattern 2-L, 4-$NR^1R^2$, 6-$NR^3$(substituted phenyl or pyridinyl) is equivalent to substitution pattern 2-L, 6-$NR^1R^2$, 4-$NR^3$(substituted phenyl or pyridinyl).

The addition salts as mentioned herein are meant to comprise the therapeutically active addition salt forms which the compounds of formula (I) or (I') are able to form with appropriate acids, such as, for example, inorganic acids such as hydrohalic acids, e.g. hydrochloric or hydrobromic acid; sulfuric; nitric; phosphoric and the like acids; or organic acids such as, for example, acetic, propanoic, hydroxyacetic, lactic, pyruvic, oxalic, malonic, succinic, maleic, fumaric, malic, tartaric, citric, methanesulfonic, ethanesulfonic, benzenesulfonic, *p*-toluenesulfonic, cyclamic, salicylic, *p*-aminosalicylic, pamoic and the like acids.

The pharmaceutically acceptable addition salts as mentioned hereinabove are also meant to comprise the therapeutically active non-toxic base, in particular, a metal or amine addition salt forms which the compounds of formula (I) or (I') are able to form. Said salts can conveniently be obtained by treating the compounds of formula (I) or (I') containing acidic hydrogen atoms with appropriate organic and inorganic bases such as, for example, the ammonium salts, the alkali and earth alkaline metal salts, e.g. the lithium, sodium, potassium, magnesium, calcium salts and the like, salts with organic bases, e.g. the benzathine, *N*-methyl-D-glucamine, hydrabamine salts, and salts with amino acids such as, for example, arginine, lysine and the like.
Conversely said salt forms can be converted by treatment with an appropriate base or acid into the free acid or base form.

The term addition salts also comprises the hydrates and the solvent addition forms which the compounds of formula (I) or (I') are able to form. Examples of such forms are e.g. hydrates, alcoholates and the like.

The term stereochemically isomeric forms of compounds of formula (I) or (I'), as used hereinbefore, defines all possible compounds made up of the same atoms bonded by the same sequence of bonds but having different three-dimensional structures which are not interchangeable, which the compounds of formula (I) or (I') may possess. Unless otherwise mentioned or indicated, the chemical designation of a compound encompasses the mixture of all possible stereochemically isomeric forms which said compound may possess. Said mixture may contain all diastereomers and/or enantiomers of the basic molecular structure of said compound. All stereochemically isomeric forms of the compounds of formula (I) or (I') both in pure form or in admixture with each other are intended to be embraced within the scope of the present invention.

Some of the compounds of formula (I) or (I') may also exist in their tautomeric forms. Such forms although not explicitly indicated in the above formula are intended to be included within the scope of the present invention.

Whenever used hereinafter, the term "compounds of formula (I) or (I')" is meant to include also the *N*-oxides, the pharmaceutically acceptable addition salts and all stereoisomeric forms.

A special group of compounds are those compounds of formula (I) or (I') wherein n is at least 1 and at least one $R^4$ is cyano; preferably, n is 1 and $R^4$ is cyano substituted in the para position relative to the $NR^3$ moiety. Said special group of compounds is deemed novel.

Another special group of compounds contains those compounds of formula (I) or (I') which are other than

(c) *N*4-methyl-*N*2-(2-methylphenyl)-*N*4-phenyl-2,4,6-pyrimidinetriamine;
(d) *N*4-methyl-*N*2-(2-methylphenyl)-*N*4-phenyl-6-(phenylmethyl)-2,4-pyrimidinediamine;
(e) *N*4-(2-methylphenyl)-6-(phenylmethyl)-2,4-pyrimidinediamine; the *N*-oxides, the pharmaceutically acceptable addition salts and the stereochemically isomeric forms thereof.

An interesting group of compounds are those compounds of formula (I) or (I') wherein the $NR^3$(substituted phenyl or pyridinyl) moiety is in the 4- or 6-position of the pyrimidine ring.

Another interesting group are those compounds of formula (I) or (I') wherein each $R^4$ independently is hydroxy, halo, $C_{1-6}$alkyloxy, cyano, aminocarbonyl, nitro, amino, trihalomethyl or trihalomethyloxy; $R^6$ is phenyl or phenyl substituted with one, two or three, four or five substituents each independently selected from halo, $C_{1-6}$alkyloxy, $C_{1-6}$alkylcarbonyl, cyano, nitro and trifluoromethyl; and aryl is phenyl or phenyl substituted with one, two, three, four or five substituents each independently selected from halo, $C_{1-6}$alkyloxy, cyano, nitro and trifluoromethyl.

Particular groups of compounds are those groups wherein one or more of the following conditions are met :

(i) n is 0 or 1;
(ii) $R^1$ and $R^2$ are each independently selected from hydrogen, hydroxy, $C_{1-12}$alkyl, $C_{1-12}$alkylcarbonyl, $C_{1-12}$alkyloxycarbonyl, cyano wherein the aforementioned $C_{1-12}$alkyl groups may optionally and each individually be substituted with one or two substituents each independently selected from hydroxy, $C_{1-6}$alkyloxy, hydroxy$C_{1-6}$alkyloxy, aryl and Het; or
$R^1$ and $R^2$ taken together may form mono- or di($C_{1-12}$alkyl)amino$C_{1-4}$alkylidene;
(iii) $R^3$ is hydrogen;
(iv) $R^4$ is cyano, halo or $C_{1-6}$alkyl;

(v) $R^5$ is hyrogen or methyl;

(vi) L is $C_{1-10}$alkyl substituted with phenyl substituted with one or two halogens; or L is -X-$R^6$ wherein $R^6$ is phenyl substituted with one or two halogens and X is -O-;

Other particular compounds are those compounds of formula (I) wherein L is 2,6-dichlorobenzyl or 2,6-dichlorophenoxy.

Still other particular compounds are those compounds of formula (I) where $R^3$ is hydrogen, A is CH, n is 1, and $R^4$ is halo, methyl or cyano and is positioned in the 4 position of the phenyl ring.

Preferred compounds are those compounds of formula (I) wherein L is 2,6-dichlorobenzyl and is in the 4 position of the pyrimidine ring, the $NR^3$(substituted phenyl or pyridinyl) moiety represents *p*-cyano-anilino and is in the 2 position of the pyrimidine ring.

In general, compounds of formula (I') can be prepared by reacting an intermediate of formula (II) wherein $W^1$ is a suitable leaving group such as, for example, a halogen, with an amino derivative of formula (III) optionally in a solvent such as, for example, 1-methyl-2-pyrrolidinone and the like under a reaction-inert atmosphere such as, for example, oxygen free argon or nitrogen, and optionally in the presence of an acid such as, for example, 1 N hydrochloric acid in diethylether.

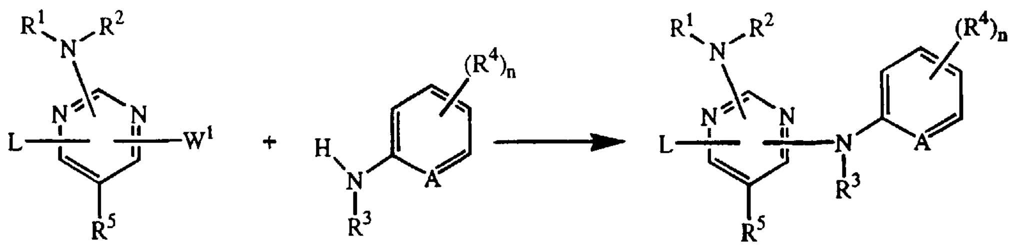

(II) (III) (I')

In this and the following preparations, the reaction products may be isolated from the reaction medium and, if necessary, further purified according to methodologies generally known in the art such as, for example, extraction, crystallization, distillation, trituration and chromatography.

In case $R^2$ contains a hydroxy moiety, it may be convenient to perform the above reaction with a protected form of intermediate (III) whereby the hydroxy moiety bears a suitable protecting group P being, for instance, a benzyl, and subsequently removing the protective group according to art-known methodologies, such as, for example, reacting with $BBr_3$ in dichloromethane under nitrogen atmosphere.

The compounds of formula (I') may further be prepared by converting compounds of formula (I') into each other according to art-known group transformation reactions.

For instance, compounds of formula (I') whereby $R^1$ and $R^2$ are taken together to form mono- or di($C_{1-12}$alkyl)amino$C_{1-4}$alkylidene said compounds being represented by formula (I'-a), may be prepared by reacting a compound of formula (I') wherein $R^1$ and $R^2$ are hydrogen, with an intermediate of formula (IV) or a functional derivative thereof.

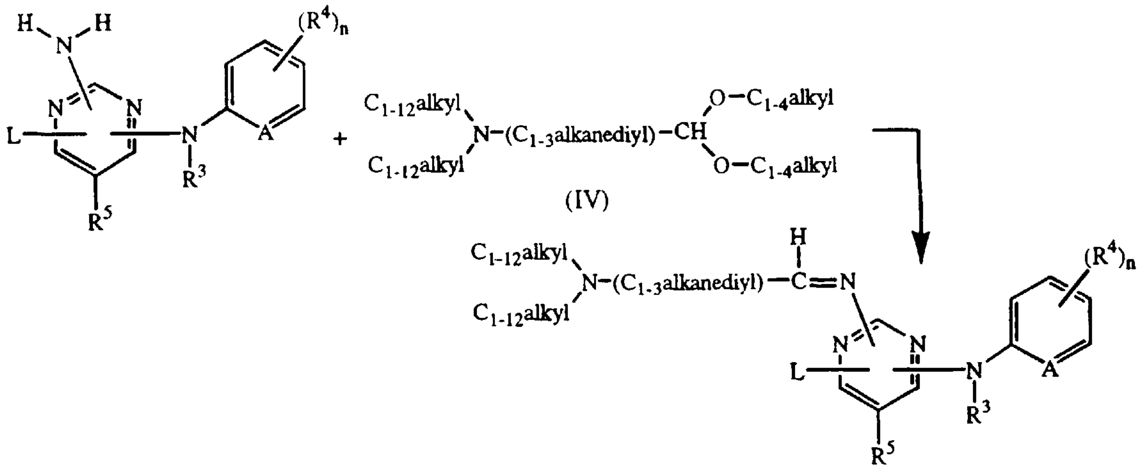

(IV)

(I'-a)

Also, compounds of formula (I') wherein $R^1$ and $R^2$ are hydrogen may further be reacted with an acyl halide or an alkyl chloroformate in a reaction-inert solvent such as, for example dichloromethane, in the presence of a suitable base, such as, for example, pyridine, to form the corresponding amide, respectively, carbamate derivative.

Some of the compounds of formula (I') and some of the intermediates in the present invention may contain an asymmetric carbon atom. Pure stereochemically isomeric forms of said compounds and said intermediates can be obtained by the application of art-known procedures. For example, diastereoisomers can be separated by physical methods such as selective crystallization or chromatographic techniques, e.g. counter current distribution, liquid chromatography and the like methods. Enantiomers can be obtained from racemic mixtures by first converting said racemic mixtures with suitable resolving agents such as, for example, chiral acids, to mixtures of diastereomeric salts or compounds; then physically separating said mixtures of diastereomeric salts or compounds by, for example, selective crystallization or chromatographic techniques, e.g. liquid chromatography and the like methods; and finally converting said separated diastereomeric salts or compounds into the corresponding enantiomers. Pure stereochemically isomeric forms may also be obtained from the pure stereochemically isomeric forms of the appropriate intermediates and starting materials, provided that the intervening reactions occur stereospecifically.

An alternative manner of separating the enantiomeric forms of the compounds of formula (I') and intermediates involves liquid chromatography, in particular liquid chromatography using a chiral stationary phase.

Some of the intermediates and starting materials are known compounds and may be commercially available or may be prepared according to art-known procedures.

Intermediates of formula (II) can be prepared by reacting a pyrimidine derivative of formula (V) wherein $W^2$ is a suitable leaving group such as, for example, a halogen, with $HNR^1R^2$ (VI) in a reaction inert solvent such as, for example, 1,4-dioxane, 2-propanol or the like. Different regio-specific isomers may be formed and can be separated from one another using suitable separation techniques such as, for example, chromatography.

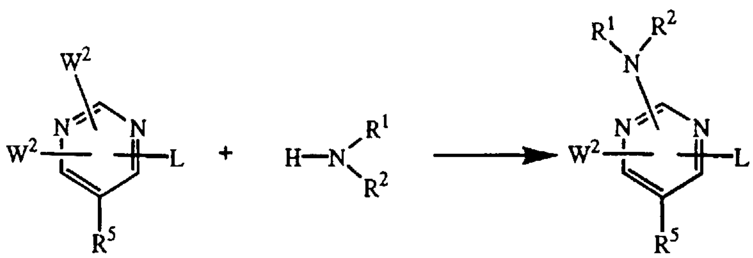

(V) (VI) (II)

Intermediates of formula (V) whereby the L is L'-$CH_2$ and is attached in the 2 position of the pyrimidine ring and $W^2$ is chloro, said intermediates being represented by formula (V-a), can be prepared by reacting an imidamide of formula (VII) with a propanedioic acid ester of formula (VIII) in a solvent such as, for example, ethanol, and in the presence

of, for instance, sodium, and subsequently reacting the thus formed intermediate of formula (IX) with a suitable reagent such as, for example, phosphoryl chloride.

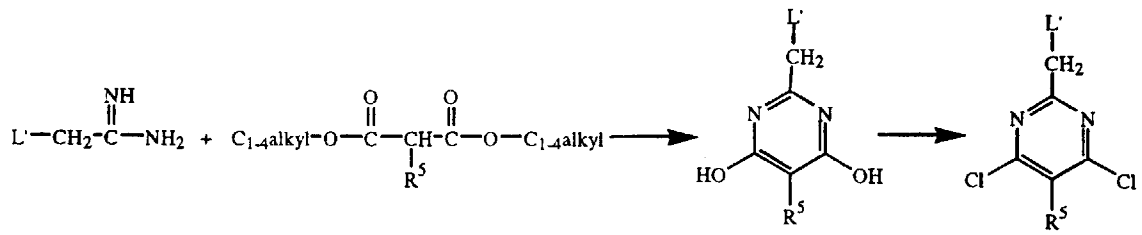

(VII) (VIII) (IX) (V-a)

Intermediates of formula (V) whereby L is L'-$CH_2$ and is attached in the 4 or 6 position of the pyrimidine ring and $W^2$ is chloro, said intermediates being represented by formula (V-b), can be prepared by reacting an intermediate of formula (X) with urea or a functional derivative thereof, in a solvent such as, for example, ethanol, and in the presence of, for instance, sodium, and subsequently reacting the thus formed intermediate of formula (XI) with a suitable reagent such as, for example, phosphoryl chloride.

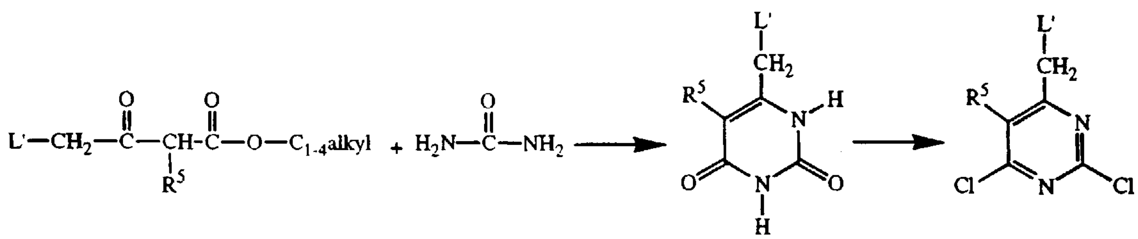

(X) (XI) (V-b)

Intermediates of formula (V) wherein L is L'-$CH_2$ and is attached anywhere on the pyrimidine ring, said intermediates being represented by formula (V-c), can be prepared by reacting an intermediate of formula (XII) wherein $W^2$ is a suitbale leaving group such as, for example, a halogen,with an intermediate of formula (XIII) wherein $W^3$ is a suitbale leaving group such as, for example, a halogen, according to the procedure of a Grignard reaction.

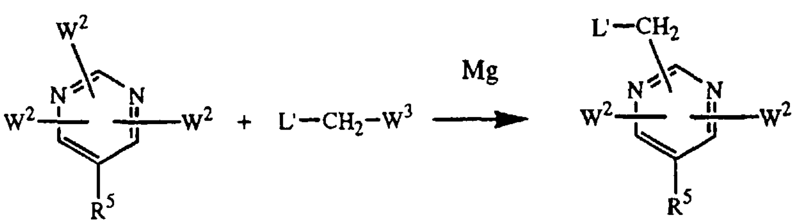


(XII) (XIII) (V-c)

Intermediates of formula (V) whereby L is O-$R^6$ and is attached in the 4 or 6 position of the pyrimidine ring, said intermediates being represented by formula (V-d), can be prepared by reacting an intermediate of formula (XIV) with an intermediate of formula (XII) wherein $W^2$ is a suitbale leaving group such as, for example, a halogen,in a reaction-inert solvent such as, for example, tetrahydrofuran, and in the presence of a suitable base such as, for example, potassium hydroxide.

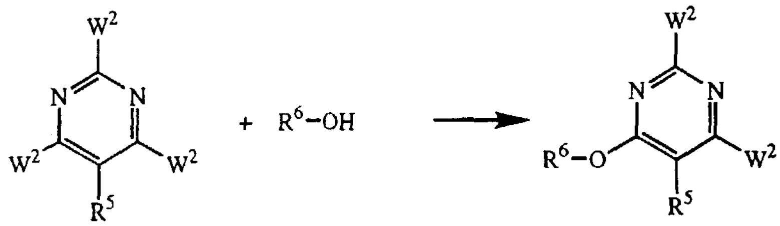

(XII) (XIV) (V-d)

Compounds of formula (I') and some of the intermediates may have one or more stereogenic centers in their structure, present in a R or a S configuration.

The compounds of formula (I') as prepared in the hereinabove described processes may be synthesized as a mixture of stereoisomeric forms, in particular in the form of racemic mixtures of enantiomers which can be separated from one another following art-known resolution procedures. The racemic compounds of formula (I) may be converted into the corresponding diastereomeric salt forms by reaction with a suitable chiral acid. Said diastereomeric salt forms are subsequently separated, for example, by selective or fractional crystallization and the enantiomers are liberated therefrom by alkali. An alternative manner of separating the enantiomeric forms of the compounds of formula (I) involves liquid chromatography using a chiral stationary phase. Said pure stereochemically isomeric forms may also be derived from the corresponding pure stereochemically isomeric forms of the appropriate starting materials, provided that the reaction occurs stereospecifically. Preferably if a specific stereoisomer is desired, said compound will be synthesized by stereospecific methods of preparation. These methods will advantageously employ enantiomerically pure starting materials.

The compounds of formula (I) and (I') show antiretroviral properties, in particular against Human Immunodeficiency Virus (HIV), which is the aetiological agent of Acquired Immune Deficiency Syndrome (AIDS) in humans. The HIV virus preferentially infects human T-4 cells and destroys them or changes their normal function, particularly the coordination of the immune system. As a result, an infected patient has an everdecreasing number of T-4 cells, which moreover behave abnormally. Hence, the immunological defense system is unable to combat infections and neoplasms and the HIV infected subject usually dies by opportunistic infections such as pneumonia, or by cancers. Other conditions associated with HIV infection include thrombocytopaenia, Kaposi's sarcoma and infection of the central nervous system characterized by progressive demyelination, resulting in dementia and symptoms such as, progressive dysarthria, ataxia and disorientation. HIV infection further has also been associated with peripheral neuropathy, progressive generalized lymphadenopathy (PGL) and AIDS-related complex (ARC).

The present compounds also show activity against HIV-1 strains that have acquired resistance to art-known non-nucleoside reverse transcriptase inhibitors. They also have little or no binding affinity to human $\alpha$-1 acid glycoprotein.

Due to their antiretroviral properties, particularly their anti-HIV properties, especially their anti-HIV-1-activity, the compounds of formula (I), their pharmaceutically acceptable salts and the stereochemically isomeric forms thereof, are useful in the treatment of individuals infected by HIV and for the prophylaxis of these individuals. In general, the compounds of the present invention may be useful in the treatment of warm-blooded animals infected with viruses whose existence is mediated by, or depends upon, the enzyme reverse transcriptase. Conditions which may be prevented or treated with the compounds of the present invention, especially conditions associated with HIV and other pathogenic retroviruses, include AIDS, AIDS-related complex (ARC), progressive generalized lymphadenopathy (PGL), as well as chronic CNS diseases caused by retroviruses, such as, for example HIV mediated dementia and multiple sclerosis.

The compounds of the present invention or any subgroup thereof may therefore be used as medicines against above-mentioned conditions. Said use as a medicine or method of treatment comprises the systemic administration to HIV-infected subjects of an amount effective to combat the conditions associated with HIV and other pathogenic retroviruses, especially HIV-1.

Apart from their pharmacological properties, the present compounds have interesting physicochemical properties. For instance, they have a good solubility.

The compounds of the present invention or any subgroup thereof may be formulated into various pharmaceutical forms for administration purposes. As appropriate compositions there may be cited all compositions usually employed for systemically administering drugs. To prepare the pharmaceutical compositions of this invention, an effective amount of the particular compound, optionally in addition salt form, as the active ingredient is combined in intimate

admixture with a pharmaceutically acceptable carrier, which carrier may take a wide variety of forms depending on the form of preparation desired for administration. These pharmaceutical compositions are desirable in unitary dosage form suitable, particularly, for administration orally, rectally, percutaneously, or by parenteral injection. For example, in preparing the compositions in oral dosage form, any of the usual pharmaceutical media may be employed such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules, and tablets. Because of their ease in administration, tablets and capsules represent the most advantageous oral dosage unit forms, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. Also included are solid form preparations which are intended to be converted, shortly before use, to liquid form preparations. In the compositions suitable for percutaneous administration, the carrier optionally comprises a penetration enhancing agent and/or a suitable wetting agent, optionally combined with suitable additives of any nature in minor proportions, which additives do not introduce a significant deleterious effect on the skin.

It is especially advantageous to formulate the aforementioned pharmaceutical compositions in dosage unit form for ease of administration and uniformity of dosage. Dosage unit form as used herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions and the like, and segregated multiples thereof.

Those of skill in the treatment of HIV-infection could determine the effective daily amount from the test results presented here. In general it is contemplated that an effective daily amount would be from 0.01 mg/kg to 50 mg/kg body weight, more preferably from 0.1 mg/kg to 10 mg/kg body weight. It may be appropriate to administer the required dose as two, three, four or more sub-doses at appropriate intervals throughout the day. Said sub-doses may be formulated as unit dosage forms, for example, containing 1 to 1000 mg, and in particular 5 to 200 mg of active ingredient per unit dosage form.

The exact dosage and frequency of administration depends on the particular compound of formula (I) used, the particular condition being treated, the severity of the condition being treated, the age, weight and general physical condition of the particular patient as well as other medication the individual may be taking, as is well known to those skilled in the art. Furthermore, it is evident that said effective daily amount may be lowered or increased depending on the response of the treated subject and/or depending on the evaluation of the physician prescribing the compounds of the instant invention. The effective daily amount ranges mentioned hereinabove are therefore only guidelines and are not intended to limit the scope or use of the invention to any extent.

Also, the combination of an antiretroviral compound and a compound of formula (I), (I') or a subgroup thereof can be used as a medicine. Thus, the present invention also relates to a product containing (a) a compound of formula (I), (I') or a subgroup thereof, and (b) another antiretroviral compound, as a combined preparation for simultaneous, separate or sequential use in anti-HIV treatment. The different drugs may be combined in a single preparation together with pharmaceutically acceptable carriers. Said other antiretroviral compounds may be known antiretroviral compounds such as nucleoside reverse transcriptase inhibitors, e.g. zidovudine (3'-azido-3'-deoxythymidine, AZT), didanosine (dideoxy inosine; ddI), zalcitabine (dideoxycytidine, ddC) or lamivudine (3'-thia-2'-3'-dideoxycytidine, 3TC) and the like; non-nucleoside reverse transciptase inhibitors such as suramine, pentamidine, thymopentin, castanospermine, dextran (dextran sulfate), foscarnet-sodium (trisodium phosphono formate), nevirapine (11-cyclopropyl-5,11-dihydro-4-methyl-6*H*-dipyrido-[3,2-b : 2',3'-e][1,4]diazepin-6-one), tacrine (tetrahydroaminoacridine) and the like; compounds of the TIBO (tetrahydro-imidazo[4,5,1-jk][1,4]-benzodiazepine-2(1*H*)-one and thione)-type e.g. (S)-8-chloro-4,5,6,7-tetrahydro-5-methyl-6-(3-methyl-2-butenyl)imidazo-[4,5,1-jk][1,4]benzodiazepine-2(1*H*)-thione; compounds of the α-APA (α-anilino phenyl acetamide) type e.g. α-[(2-nitro-phenyl)amino]-2,6-dichlorobenzeneacetamide and the like; TAT-inhibitors, e.g. RO-5-3335 and the like; protease inhibitors e.g. indinavir, ritanovir, saquinovir and the like; or immunomodulating agents, e.g. levamisole and the like.

The following examples are intended to illustrate the present invention.

Experimental part

A. Intermediate compounds

Example A1

a) A solution of 2,6-dichlorobenzylchloride (0.102 mol) in 1,1-diethylether (10 ml) was added dropwise to magnesium (0.102 mol) in 1,1-diethylether (60 ml). The reaction was initiated by adding 2 drops of 1,2-dibromoethane. After most of magnesium disappeared, 2,4,6-trichloropyrimidine (0.051 mol) in 1,1-diethylether (30 ml) was added. The mixture was stirred overnight at room temperature. The solvent was evaporated and the residue was purified by flash column chromatography over silica gel (eluent: $CH_2Cl_2$/hexane 1/2). The desired fractions were collected and the solvent was evaporated, yielding 3.3 g of (21%) 2,4-dichloro-6-[(2,6-dichlorophenyl) methyl]pyrimidine (interm. 1; m.p.: 106-107 °C).

b) Intermediate (1) (0.0081 mol) in 2-propanol (100 ml) was heated until complete dissolution. The solution was then transferred into a pressure tube and $NH_3$ gas was bubbled into it for 20 minutes. Then the mixture was heated to 80 °C for 16 hours. The solvent was evaporated, yielding a residue of two compounds : 2-chloro-6-[(2,6-dichlorophenyl)methyl]-4-pyrimidinamine (interm. 2) and 4-chloro-6-[(2,6-dichlorophenyl)methyl]-2-pyrimidinamine (interm. 3).

Example A2

a) Urea (0.03 mol) was added to a mixture of (±)-ethyl 2,6-dichlorophenyl-α-methyl-β-oxobenzenebutanoate (0.02 mol) in $NaOC_2H_5$, (1M; 40 ml) and ethanol (10 ml). The reaction mixture was stirred and refluxed overnight. The solvent was evaporated, water was added and the mixture was neutralized with 0.3 N HOAc. The precipitate was filtered off and was further triturated with ether and then $H_2O$, then filtered off and dried, yielding 2.2 g (39%) of 6-[(2,6-dichlorophenyl)methyl]-5-methyl-2,4(1*H*,3*H*)-pyrimidinedione (interm. 4).

b) A mixture of intermediate (4) (0.0095 mol) in phosphoryl chloride (50 ml) was stirred and refluxed overnight. Excess of phosphoryl chloride was then evaporated. Ice-water was added to the residue. A white precipitate was formed, filtered off and dried. The residue was purified by flash column chromatography over silica gel (eluent: $CH_2Cl_2$). The desired fractions were collected and the solvent was evaporated, yielding 2.06 g (67%) of 2,4-dichloro-6-[(2,6-dichlorophenyl)methyl]-5-methylpyrimidine (interm. 5).

c) 4-chloro-6-[(2,6-dichlorophenyl)methyl]-5-methyl-2-pyrimidinamine (interm. 6) and 2-chloro-6-[(2,6-dichlorophenyl)methyl]-5-methyl-4-pyrimidinamine (interm. 7) were prepared following the procedures as described in example A1b.

Example A3

a) To the stirred solution of 2,6-dichlorobenzeneethanimidamide HCl (1:1), (0.0042 mol) in ethanol (20 ml), a solution of sodium (0.013 mol) in ethanol (10 ml) was added dropwise first and then propanedioic acid, diethyl ester (0.0109 mol) was added. The reaction mixture was stirred and refluxed for 4 hours and then stirred at room temperature overnight After adding another equivalent of propanedioic acid, diethyl ester (stirring and refluxing it overnight) and adding more propanedioic acid, diethyl ester (0.34 g) (stirring and refluxing it overnight), the solvent was evaporated and the residue was dissolved in water and acidified with 1 N HCl. The solid was filtered off, washed with water and dried, yielding 0.87 g (76.4%) of 2-[(2,6-dichlorophenyl)methyl]-4,6-pyrimidinediol (interm. 8).

b) 6-chloro-2-[(2,6-dichlorophenyl)methyl]-4-pyrimidinamine (interm. 9) was prepared staring from intermediate according to the procedures described in example A2.b & A2.c.

Example A4

4-Amino-1-butanol (1.57 ml) was added to a solution of intermediate (1) (0.008 mol) in 1,4-dioxane (20 ml) under Argon. The reaction mixture was stirred for 2 hours at room temperature. The solvent was evaporated. The residue was purified by flash column chromatography over silica gel (eluent gradient $CH_2Cl_2$/$CH_3OH$: from 100/0 to 98/2).

The pure fractions were collected and the solvent was evaporated, yielding 2.05 g of a mixture of 4-[[2-chloro-6-[(2,6-dichlorophenyl)methyl]-4-pyrimidinyl]amino]-1-butanol (interm. 10) and 4-[[4-chloro-6-[(2,6-dichlorophenyl)methyl]-2-pyrimidinyl]amino]-1-butanol (interm. 11).

Example A5

Potassium hydroxide/ethanol (10%; 0.035 mol) was added to a solution of 2,6-phenol (0.035 mol) in tetrahydrofuran (100 ml). The mixture was stirred and 2,4,6-pyrimidine (0.044 mol) was added. The mixture was stirred overnight at 60 °C. The reaction was quenched with NaOH 1N solution. The aqueous layers were extracted with EtOAc several times and then the organic layers were combined and washed with NaOH 3N and saturated NaCl, dried and concentrated. The residue was recrystallized from $CH_2Cl_2$/hexane. The precipitate was filtered off and dried, yielding 5.98 g 2,4-dichloro-6-(2,6-dichlorophenoxy)pyrimidine (55%) (interm. 12).

Example A6

Pyridine (1 ml) was added to a mixture of 4-[[4-amino-6-[(2,6-dichlorophenyl)methyl]-2-pyrimidinyl]amino]benzonitrile (0.00135 mol) in $CH_2Cl_2$ (19 ml). A solution of chloroethanoyl chloride (0.001375 mol) in $CH_2Cl_2$ (0.5 ml) was added dropwise on an ice bath. The mixture was stirred at room temperature for 2 hours. More chloroethanoyl chloride (0.00625 mol) in $CH_2Cl_2$ (0.5 ml) was added. The mixture stood in the refrigerator overnight. The solvent was evaporated. The residue was treated with a saturated $Na_2CO_3$ solution and the mixture was extracted with $CH_2Cl_2$. The separated organic layer was dried, filtered and concentrated. The residue was purified by flash column chromatography over silica gel (eluent: $CH_2Cl_2/CH_3OH/NH_4OH$ 99/1/0.1). The desired fractions were collected and the solvent was evaporated, yielding 0.22 g (36.5%) of 2-chloro-*N*-[6-[(2,6-dichlorophenyl)methyl]-2-[(4-cyanophenyl)amino]-4-pyrimidinyl]acetamide (interm. 13).

Example A7

A mixture of 2-chloro-6-[(2,6-dichlorophenyl)methyl]-*N*-(phenylmethoxy)-4-pyrimidinamine (0.01 mol) and 4-aminobenzonitrile (0.022 mol) in 1-methyl-2-pyrrolidinone (4 ml) was stirred at 100 °C for 16 hours under nitrogen in a sealed pressure tube. The crude reaction mixture was taken up in minimal boiling EtOAc. Ether was added and the mixture was allowed to cool. The resulting precipitate was collected on a fritted Buchner funnel. The mother liquor was concentrated and used to collect a second crop of product by the same method, yielding 4.02 g (84%) of 4-[[4-[(2,6-dichlorophenyl)methyl]-6-[(phenylmethoxy)amino]-2-pyrimidinyl]amino]benzonitrile (interm. 14).

Tables 1 and 2 list intermediates which were prepared according to one of the above examples.

Table 1

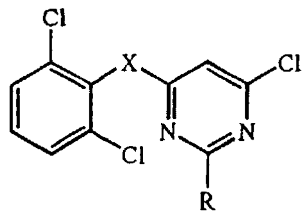

| Int. No. | Ex. No. | X | R | physical data melting point in °C |
|---|---|---|---|---|
| 15 | A1b | $CH_2$ | $-NH-CH_3$ | - |
| 16 | A1b | O | $-NH-CH_3$ | 152-155°C |
| 17 | A1b | O | $-NH_2$ | - |
| 19 | A3 | $CH_2$ | $-NH-(CH_2)_3-OH$ | - |
| 20 | A3 | $CH_2$ | $-NH-(CH_2)_2-OH$ | 111-113°C |
| 21 | A3 | $CH_2$ | $-NH-CH_2-CH(OH)-C_6H_5$ | 133-134°C |
| 22 | A3 | $CH_2$ | 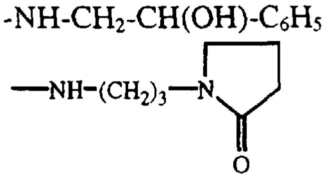 | - |
| 23 | A3 | $CH_2$ | $-NH-(CH_2)_2-O-(CH_2)_2OH$ | 99-107°C |
| 24 | A3 | $CH_2$ | $-NH-(CH_2)_2-(4-OCH_3-C_6H_4)$ | 138-140°C |
| 25 | A3 | $CH_2$ | $-NH-(CH_2)_2-(3-OCH_3-C_6H_4)$ | 132-135°C |
| 26 | A3 | $CH_2$ | $-NH-CH_2-CH(OH)-CH_2OH$ | 116-118°C |
| 27 | A3 | $CH_2$ | $-NH-CH_2-C_6H_5$ | 137-139°C |
| 28 | A3 | $CH_2$ | $-NH-(CH_2)_2-$(2-thienyl) | 113-114°C |
| 29 | A3 | $CH_2$ | $-NH-(CH_2)_2-$(2-pyridinyl) | 113.5-114°C |
| 31 | A3 | $CH_2$ | $-NH-(CH_2)_2CN$ | 151-153°C |

Table 2

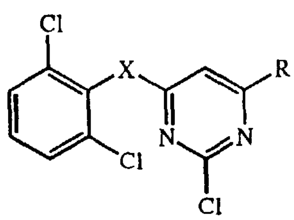

| Int. No. | Ex. No. | X | R | physical data |
|---|---|---|---|---|
| 32 | A1b | $CH_2$ | $-NH-CH_3$ | - |
| 33 | A3 | $CH_2$ | $-NH-(CH_2)_2-$(1-pyrrolidinyl) | 134-135°C |
| 34 | A3 | $CH_2$ | $-NH-(CH_2)_2-$(2-pyridinyl) | 130-133°C |
| 35 | A3 | $CH_2$ | $-NH-(CH_2)_2-$(2-thienyl) | 98-99°C |
| 36 | A3 | $CH_2$ | $-NH-(CH_2)_2-(3-OCH_3-C_6H_4)$ | 104-109°C |
| 37 | A3 | $CH_2$ | $-NH-(CH_2)_2-(4-OCH_3-C_6H_4)$ | 149-150°C |
| 38 | A3 | $CH_2$ | $-NH-(CH_2)_2CN$ | 137-139°C |
| 39 | A3 | $CH_2$ | $-NH-(CH_2)_2-O-(CH_2)_2OH$ | - |
| 40 | A3 | $CH_2$ | $-NH-(CH_2)_2OH$ | 170-173°C |
| 41 | A3 | $CH_2$ | $-NH-(CH_2)_3-O-CH(CH_3)_2$ | - |
| 42 | A3 | $CH_2$ | $-NH-(CH_2)_3-OH$ | - |
| 43 | A3 | $CH_2$ | $-NH-CH_2-C_6H_5$ | 171-172°C |
| 45 | A3 | $CH_2$ | $-NH-CH_2-CH(OH)-CH_2OH$ | >60°C |
| 46 | A3 | $CH_2$ | $-NH-O-CH_2-C_6H_5$ | 137-141°C |
| 47 | A3 | $CH_2$ | 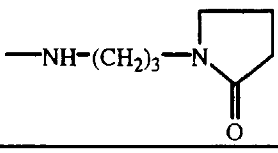 | 55-60°C |

B. Compounds of formula (I')

Example B1

A mixture of intermediate (42) and intermediate (19) (0.004 mol) and 4-aminobenzonitrile (0.0084 mol) were combined in a sealed tube and heated for 16 hours at 160 °C under Argon. The reaction mixture was allowed to cool to room temperature and dissolved in $CH_2Cl_2/CH_3OH$ 90/10 (20 ml) and 5 g of silica gel was added. After evaporating the solvent, the residue was purified by flash column chromatography over silica gel (eluent gradient: $CH_2Cl_2/CH_3OH$: from 100/0 to 97/3). The desired fraction was collected and the solvent was evaporated, yielding 0.31 g (18.1%) of 4-[[4-[(2,6-dichloropheny)methyl]-6-[(3-hydroxypropyl)amino]-2-pyrimidinyl]amino]benzonitrile (compound 3; mp. 91-105°C).

Example B2

Intermediates (47) and (22) (0.00399 mol) and 4-aminobenzonitrile (0.0012 mol) in 1-methyl-2-pyrrolidinone (3 ml) was stirred for 16 hours at 130 °C under Argon. Then, the reaction mixture was cooled to room temperature and quenched with $H_2O$ (200 ml).

A precipitate formed, which was stirred for 16 hours, and separated by filtration over Celite. The residue was dissolved in $CH_3OH/CH_2Cl_2$ (10%, 200 ml), dried over $K_2CO_3$, filtered, and evaporated. This resulting material was further purified by flash column chromatography over silica gel (gradient eluent: $CH_2Cl_2/CH_3OH$ from 100/0 to 95/5). The desired fraction was collected and the solvent was evaporated, yielding 0.43 g (21.7%) of 4-[[6-[(2,6-dichlorophenyl)methyl]-2-[[3-(2-oxo-1-pyrrolidinyl)propyl]amino]-4-pyrimidinyl]amino]benzonitrile (comp. 39; 104-114°C).

Example B3

HCl/diethyl ether (1N; 2.77 ml) was stirred into a solution of intermediate (33) (0.00277 mol) in 1-methyl-2-pyrrolidinone (4 ml) under $N_2$ atmosphere. The reaction mixture was heated for 5 minutes. Next, 4-aminobenzonitrile (0.0061 mol) was added and the reaction was heated at 100 °C for 16 hours. Then, the reaction mixture was cooled to room temperature and diluted with ethylacetate (10 ml). The organic layer was washed with NaOH (1 N; 2 x 100 ml), $H_2O$ (2 x 100 ml), brine (50 ml), respectively, dried, filtered and the filtrate was evaporated. The crude material was purified by flash chromatography (eluent: 2.5-7.5% of $CH_3OH$ containing 10% $NH_4OH$ in $CH_2Cl_2$). The desired fractions were collected and the solvent was evaporated. The residue was dried , yielding 0.160 g (12.0%) of 4-[[4-[(2,6-dichlorophenyl)methyl]-6-[[2-(1-pyrrolidinyl)ethyl]amino]-2-pyrimidinyl]amino]benzonitrile (comp. 13; mp. 80-85°C).

Example B4

A slurry of intermediate (14) (0.005 mol) in $CH_2Cl_2$ (150 ml) was stirred rapidly and cooled to 0 °C under nitrogen. $BBr_3$ (0.015 mol) was introduced by syringe. The reaction mixture was stirred rapidly for two hours. The reaction mixture was recooled to 0 °C and quenched with NaOH (aq. 1 N, 25 ml). The biphasic partial quench mixture gives a precipitate which was filtered off and dried, yielding 2.5 g (91%) of 4-[[4-[(2,6-dichlorophenyl)methyl]-6-(hydroxyamino)-2-pyrimidinyl]amino]benzonitrile dihydrobromide.pentahydrate (comp. 15; mp. 240-244°C).

Example B5

B (0.152 mol) was added to 4-[[4-amino-6-[(2,6-dichlorophenyl)methyl]-2-pyrimidinyl]amino]benzonitrile (0.0008 mol). The mixture was stirred at room temperature for 2 days and then concentrated. The crude product was purified by flash chromatography over silica gel (eluent: $CH_2Cl_2/CH_3OH$ 99/1). The desired fraction was collected and the solvent was evaporated. The resulting residue was triturated with hexane, yielding 0.15 g (42%) of *N'*-[2-[(4-cyanophenyl)amino]-6-[(2,6-dichlorophenyl)methyl]-4-pyrimidinyl]-*N*,*N*-dimethylmethanimidamide (comp. 26; mp. 175-180°C).

Example B6

Piperidine (0.12 ml) was added to a mixure of intermediate (13) (0.00047 mol) in terahydrofuran (20 ml). The mixture was stirred at room temperature for 4 hours. More piperidine (0.14 ml) was added. The mixture was stirred for another 2 hours. The solvent was evaporated. The residue was purified by flash column chromatography over silica gel ($CH_2Cl_2/CH_3OH/NH_4OH$ 99/1/0.1). The desired fractions were collected and the solvent was evaporated, yielding 0.05 g (21.5%) of N-[6-[(2,6-dichlorophenyl)methyl]-2-[(4-cyanophenyl)amino]-4-pyrimidinyl]-1-piperidineacetamide (comp. 25; mp. 175-180°C).

Example B7

Pyridine (0.014 mol) was added to a mixture of 4-[[4-amino-6-[(2,6-dichlorophenyl)methyl]-2-pyrimidinyl]amino]benzonitrile (0.0013 mol) in $CH_2Cl_2$. A solution of octanoyl chloride (1.5 equiv) in $CH_2Cl_2$ (0.5 ml) was added dropwise. The mixture was stirred at room temperature for 2 hours. More octanoyl chloride (3.5 equiv) in $CH_2Cl_2$ was added dropwise. The mixture was stirred. The solvent was then evaporated. The residue was treated with a saturated aqueous $NaHCO_3$ solution and the mixture was extracted with $CH_2Cl_2$. The separated organic layer was dried, filtered and the solvent was evaporated to give the crude product. The residue was recrystallized from $CHCl_3$ and hexane, yielding 0.443 g (68.6%) of N-[6-[(2,6-dichlorophenyl)methyl]-2-[(4-cyanophenyl)amino]-4-pyrimidinyl]octanamide (comp. 17; mp. 135-137°C).

Tables 3, 4 and 5 list the compounds of formula (I) that were prepared according to one of the above examples.

Table 3

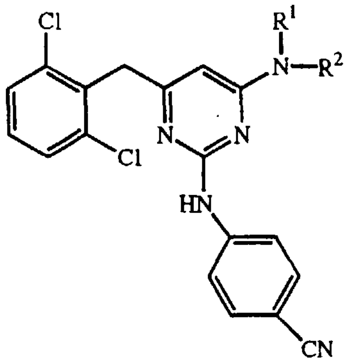

| Co. No. | Ex. No. | $NR^1R^2$ | physical data (melting point in °C) |
|---|---|---|---|
| 1 | B2 | $-NH-(CH_2)_4-OH$ | 161-163°C |
| 2 | B2 | $-NH-(CH_2)_2-OH$ | 207-210°C |
| 3 | B2 | $-NH-(CH_2)_3-OH$ | 152-154°C |
| 4 | B2 | $-NH-CH_2-CHOH-C_6H_5$ | 158-165°C |
| 5 | B2 | 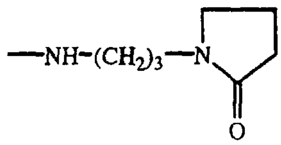 | 48-56°C |
| 6 | B2 | $-NH-(CH_2)_2-O-(CH_2)_2-OH$ | 162-175°C; HCl (1:1) |
| 7 | B2 | $-NH-(CH_2)_3-O-CH(CH_3)_2$ | 181-182°C; HCl (1:1) |
| 8 | B2 | $-NH-(CH_2)_2-(3-OCH_3-C_6H_4)$ | 72-80°C |
| 9 | B2 | $-NH-CH_2-CHOH-CH_2OH$ | 189-192°C |
| 10 | B2 | $-NH-(CH_2)_2-(4-OCH_3-C_6H_4)$ | 72-80°C |
| 12 | B2 | $-NH-CH_2-C_6H_5$ | - |
| 13 | B3 | $-NH-(CH_2)_2-$(1-pyrrolidinyl) | 80-85°C |
| 14 | B2 | $-NH-(CH_2)_2-$(2-thienyl) | - |
| 15 | B4 | -NH-OH | 240-244°C |
| 16 | B2 | $-NH-(CH_2)_2-$(2-pyridinyl) | 75-80°C |
| 17 | B7 | $-NH-CO-C_7H_{15}$ | 135-137°C |
| 18 | B7 | $-NH-CO-C_{11}H_{23}$ | 130-135°C |
| 19 | B2 | $-NH-(CH_2)_2-CN$ | 255°C; HCl (1:1) |
| 20 | B7 | $-NH-CO-O-C_2H_5$ | >200°C |
| 21 | B7 | $-NH-CO-CH_3$ | 128-130°C |
| 22 | B7 | $-NH-CO-C_3H_7$ | >200°C |
| 23 | B1 | $-NH_2$ | 94-97°C |
| 24 | B1 | $-NH-CH_3$ | 178-180°C |
| 25 | B6 | $-NH-CO-CH_2-$(1-piperidinyl) | 175-180°C |
| 26 | B5 | $-N=CH-N(CH_3)_2$ | 175-180°C |

Table 4

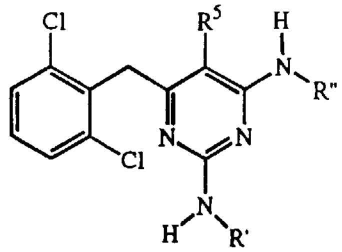

| | Co. No. | Ex. No. | R' | R'' | $R^5$ | physical data (melting point) |
|---|---|---|---|---|---|---|
| 129.324 | 27 | B1 | 4-Br-$C_6H_4$ | H | H | - |
| 129.323 | 28 | B1 | H | 4-Br-$C_6H_4$ | H | - |
| 129.326 | 29 | B1 | 4-Cl-$C_6H_4$ | H | H | - |
| 129.325 | 30 | B1 | H | 4-Cl-$C_6H_4$ | H | - |
| 129.936 | 31 | B1 | H | (3-Br-6-pyridinyl) | H | - |
| 129.937 | 32 | B1 | (3-Br-6-pyridinyl) | H | H | - |
| 129.947 | 33 | B1 | 4-F-$C_6H_4$ | H | H | 77-80°C |
| 129.948 | 34 | B1 | H | 4-F-$C_6H_4$ | H | >200°C |
| 129.949 | 35 | B1 | 4-$CH_3$-$C_6H_4$ | H | H | 76-79°C |
| 129.950 | 36 | B1 | H | 4-$CH_3$-$C_6H_4$ | H | 183-186°C |
| 129.951 | 37 | B1 | $C_6H_5$ | H | H | 85-90°C |
| 129.952 | 38 | B1 | H | $C_6H_5$ | H | 182-187°C |
| 132.536 | 39 | B2 | 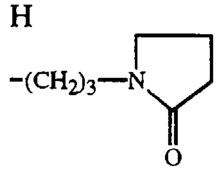 | 4-CN-$C_6H_4$ | H | 104-114°C |
| 135.095 | 40 | B2 | $(CH_2)_2$-OH | 4-CN-$C_6H_4$ | H | 247-250°C; HCl (1:1) |
| 132.291 | 41 | B1 | $CH_3$ | 4-CN-$C_6H_4$ | H | >200°C |
| 131.646 | 42 | B1 | $(CH_2)_3$-OH | 4-CN-$C_6H_4$ | H | 91-105°C |
| 131.113 | 43 | B2 | $(CH_2)_4$-OH | 4-CN-$C_6H_4$ | H | 161-163°C |
| 126.875 | 45 | B1 | H | 4-CN-$C_6H_4$ | H | >200°C |
| 137.257 | 46 | B1 | H | 4-CN-$C_6H_4$ | $CH_3$ | >200°C |
| 137.258 | 47 | B1 | 4-CN-$C_6H_4$ | H | $CH_3$ | >200°C |
| 137.259 | 48 | B1 | H | 4-Br-$C_6H_4$ | $CH_3$ | >200°C |
| 137.260 | 49 | B1 | 4-Br-$C_6H_4$ | H | $CH_3$ | 168-170°C |

Table 5

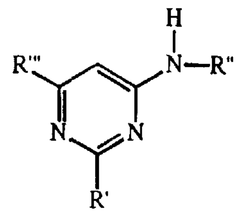

| Co. No. | Ex. No. | R' | R" | R''' | physical data (melting point) |
|---|---|---|---|---|---|
| 50 | B1 | $NH_2$ | 4-CN-$C_6H_4$ | O-(2,6-diCl-$C_6H_3$) | >200°C |
| 51 | B1 | $CH_2$-(2,6-diCl-$C_6H_3$) | H | -NH-(4-CN-$C_6H_4$) | >200°C |

C. Pharmacological example

Example C.1

A rapid, sensitive and automated assay procedure was used for the *in vitro* evaluation of anti-HIV agents. An HIV-1 transformed T4-cell line, MT-4, which was previously shown (Koyanagi et al., *Int. J. Cancer*, 36, 445-451, 1985) to be highly susceptible to and permissive for HIV infection, served as the target cell line. Inhibition of the HIV-induced cytopathic effect was used as the end point. The viability of both HIV- and mock-infected cells was assessed spectrophotometrically via the *in situ* reduction of 3-(4,5-dimethylthiazol-2-yl)-2,5-diphenyltetrazolium bromide (MTT). The 50% cytotoxic concentration ($CC_{50}$ in μM) was defined as the concentration of compound that reduced the absorbance of the mock-infected control sample by 50%. The percent protection achieved by the compound in HIV-infected cells was calculated by the following formula :

$$\frac{(OD_T)_{HIV}-(OD_C)_{HIV}}{(OD_C)_{MOCK}-(OD_C)_{HIV}} \quad \text{expressed in \%,}$$

whereby $(OD_T)_{HIV}$ is the optical density measured with a given concentration of the test compound in HIV-infected cells; $(OD_C)_{HIV}$ is the optical density measured for the control untreated HIV-infected cells; $(OD_C)_{MOCK}$ is the optical density measured for the control untreated mock-infected cells; all optical density values were determined at 540 nm. The dose achieving 50% protection according to the above formula was defined as the 50% inhibitory concentration ($IC_{50}$ in μM). The ratio of $CC_{50}$ to $IC_{50}$ was defined as the selectivity index (SI). The compounds of formula (I) were shown to inhibit HIV-1 effectively. Particular $IC_{50}$, $CC_{50}$ and SI values are listed in Table 6 hereinbelow.

Table 6

| Co. No. | $IC_{50}$ (μM | $CC_{50}$ (μM | SI | | Co. No. | $IC_{50}$ (μM | $CC_{50}$ (μM | SI |
|---|---|---|---|---|---|---|---|---|
| 1 | 0.027 | 49.7 | 1860 | | 27 | 0.017 | >20 | >1169 |
| 2 | 0.035 | >100 | >2890 | | 28 | 0.079 | >20 | >253 |
| 3 | 0.016 | 37.4 | 2558 | | 29 | 0.015 | >20 | >1324 |
| 4 | 0.315 | >100 | >317 | | 30 | 0.088 | >20 | >228 |
| 5 | 0.094 | 56.2 | 598 | | 31 | 0.024 | 86.8 | 3630 |
| 6 | 0.020 | 24.4 | 1192 | | 32 | 0.403 | >100 | >248 |

Table 6 (continued)

| Co. No. | $IC_{50}$ (μM | $CC_{50}$ (μM | SI | | Co. No. | $IC_{50}$ (μM | $CC_{50}$ (μM | SI |
|---|---|---|---|---|---|---|---|---|
| 7 | 0.975 | >100 | >102 | | 33 | 0.042 | 43.4 | 1038 |
| 8 | 8.147 | >100 | >12 | | 34 | 0.319 | 57.8 | 181 |
| 9 | 0.037 | 58.6 | 1587 | | 35 | 0.103 | 42.3 | 409 |
| 10 | 2.529 | >100 | >39 | | 36 | 0.323 | >100 | >309 |
| 12 | 1.683 | 55.1 | 32 | | 37 | 0.443 | 33.4 | 75 |
| 13 | 0.005 | 7.8 | 1557 | | 38 | 2.449 | 32.4 | 13 |
| 14 | 2.183 | 89.0 | 40 | | 39 | 1.531 | >100 | >65 |
| 15 | 0.003 | 9.0 | 2857 | | 40 | 0.253 | 40.2 | 158 |
| 16 | 0.389 | 41.2 | 105 | | 41 | 1.986 | 34.2 | 17 |
| 17 | 0.167 | 9.1 | 54 | | 42 | 0.352 | 35.5 | 88 |
| 18 | 2.056 | 59.9 | 29 | | 43 | 0.603 | >100 | >165 |
| 19 | 0.006 | 53.6 | 8642 | | 45 | 0.010 | 56.3 | 5688 |
| 20 | 0.026 | 36.5 | 1413 | | 46 | 45.186 | >100 | >2 |
| 21 | 0.017 | 50.6 | 2910 | | 47 | 0.004 | >100 | >27027 |
| 22 | 0.035 | 12.2 | 346 | | 48 | 44.157 | >100 | >1 |
| 23 | 0.001 | 47.9 | 59935 | | 49 | 0.058 | 45.2 | 786 |
| 24 | 0.020 | 54.0 | 2667 | | 50 | 0.518 | 52.0 | 100 |
| 25 | 0.079 | >100 | >1272 | | 51 | 0.452 | >100 | >221 |
| 26 | 0.011 | 33.5 | 2990 | | | | | |

## Claims

1. The use of a compound of formula

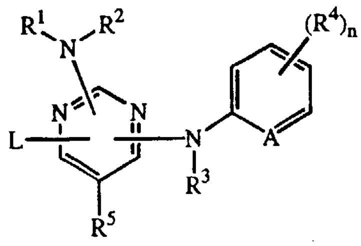

(I)

a *N*-oxide, a pharmaceutically acceptable addition salt or a stereochemically isomeric form thereof, wherein

A is CH or N;
n is 0, 1, 2, 3 or 4; and in case A is CH, then n may also be 5;
$R^1$ and $R^2$ are each independently selected from hydrogen, hydroxy, $C_{1-12}$alkyl, $C_{1-12}$alkyloxy, $C_{1-12}$alkylcarbonyl, $C_{1-12}$alkyloxycarbonyl, aryl, amino, mono- or di($C_{1-12}$alkyl)amino, mono- or di($C_{1-12}$alkyl)aminocarbonyl wherein each of the aforementioned $C_{1-12}$alkyl groups may optionally and each individually be substituted with one or two substituents each independently selected from hydroxy, $C_{1-6}$alkyloxy, hydroxy$C_{1-6}$alkyloxy, carboxyl, $C_{1-6}$alkyloxycarbonyl, cyano, amino, imino, aminocarbonyl, aminocarbonylamino, mono- or di($C_{1-6}$alkyl)amino, aryl and Het; or
$R^1$ and $R^2$ taken together may form pyrrolidinyl, piperidinyl, morpholinyl, azido or mono- or di($C_{1-12}$alkyl)amino$C_{1-4}$alkylidene;

$R^3$ is hydrogen, aryl, $C_{1-6}$alkylcarbonyl, $C_{1-6}$alkyl, $C_{1-6}$alkyloxycarbonyl, $C_{1-6}$alkyl substituted with $C_{1-6}$alkyloxycarbonyl; and

each $R^4$ independently is hydroxy, halo, $C_{1-6}$alkyl, $C_{1-6}$alkyloxy, cyano, aminocarbonyl, nitro, amino, trihalomethyl or trihalomethyloxy ;

$R^5$ is hydrogen or $C_{1-4}$alkyl;

L is $C_{1-10}$alkyl, $C_{3-10}$alkenyl, $C_{3-10}$alkynyl, $C_{3-7}$cycloalkyl, or $C_{1-10}$alkyl substituted with one or two substituents independently selected from

* $C_{3-7}$cycloalkyl,
* indolyl or indolyl substituted with one, two, three or four substituents each independently selected from halo, $C_{1-6}$alkyl, $C_{1-6}$alkyloxy, cyano, aminocarbonyl, nitro, amino, trihalomethyl, trihalomethyloxy and $C_{1-6}$alkylcarbonyl,
* phenyl or phenyl substituted with one, two, three, four or five substituents each independently selected from halo, hydroxy, $C_{1-6}$alkyl, $C_{1-6}$alkyloxy, cyano, aminocarbonyl, nitro, amino, trihalomethyl, trihalomethyloxy and $C_{1-6}$alkylcarbonyl; or

L is -X-$R^6$ wherein

$R^6$ is phenyl or phenyl substituted with one, two, three, four or five substituents each independently selected from halo, $C_{1-6}$alkyl, $C_{1-6}$alkyloxy, $C_{1-6}$alkylcarbonyl, cyano, nitro and trifluoromethyl; and
X is -$NR^3$-, -NH-NH-, -N=N-, -O-, -S-, -S(=O)- or -$S(=O)_2$-;

aryl is phenyl or phenyl substituted with one, two, three, four or five substituents each independently selected from halo, $C_{1-6}$alkyl, $C_{1-6}$alkyloxy, cyano, nitro and trifluoromethyl;

Het is an aliphatic or aromatic heterocyclic radical; said aliphatic heterocyclic radical is selected from pyrrolidinyl, piperidinyl, homopiperidinyl, piperazinyl, morpholinyl, tetrahydrofuranyl and tetrahydrothienyl wherein each of said aliphatic heterocyclic radical may optionally be substituted with an oxo group; and said aromatic heterocyclic radical is selected from pyrrolyl, furanyl, thienyl, pyridinyl, pyrimidinyl, pyrazinyl and pyridazinyl wherein each of said aromatic heterocyclic radical may optionally be substituted with hydroxy;

for the manufacture of a medicine for the treatment of subjects suffering from HIV (Human Immunodeficiency Virus) infection.

2. The use of a compound as claimed in claim 1 wherein n is at least 1 and at least one $R^4$ is cyano.

3. The use of a compound as claimed in claim 1 or 2 wherein the $NR^3$(substituted phenyl or pyridinyl) moiety is in the 4- or 6-position of the pyrimidine ring.

4. The use of a compound as claimed in any one of claims 1 to 3 wherein L is 2,6-dichlorobenzyl and is in the 4 position of the pyrimidine ring, the $NR^3$(substituted phenyl or pyridinyl) moiety represents *p*-cyano-anilino and is in the 2 position of the pyrimidine ring.

5. A compound of formula

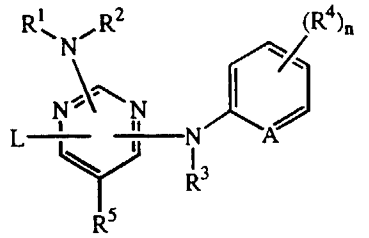

(I')

wherein $R^1$ to $R^5$, n and A are as defined in claim 1;

L is $C_{1-10}$alkyl substituted with one or two substituents independently selected from phenyl or phenyl substituted with one, two, three, four or five substituents each independently selected from halo, hydroxy, $C_{1-6}$alkyl, $C_{1-6}$alkyloxy, cyano, aminocarbonyl, nitro, amino, trihalomethyl, trihalomethyloxy and $C_{1-6}$alkylcarbonyl; or L is -X-$R^6$ wherein

$R^6$ is phenyl or phenyl substituted with one, two, three, four or five substituents each independently selected from halo, $C_{1-6}$alkyl, $C_{1-6}$alkyloxy, $C_{1-6}$alkylcarbonyl, cyano, nitro and trifluoromethyl; and

X is -$NR^3$-, -NH-NH-, -N=N-, -O-, -S-, -S(=O)- or -$S(=O)_2$-; X is -$NR^3$-, -NH-NH-, -N=N-, -O-, -S-, -S(=O)- or -$S(=O)_2$-; with the proviso that compounds

(a) *N*2-hydroxy-*N*2-methyl-*N*4,*N*6-diphenyl-2,4,6-pyrimidinetriamine;
(b) *N*,*N*,*N*',*N*',*N*'',*N*''-hexakis(3-methylphenyl)-2,4,6-pyrimidinetriamine;
(c) *N*4-methyl-*N*2-(2-methylphenyl)-*N*4-phenyl-2,4,6-pyrimidinetriamine;
(d) *N*4-methyl-*N*2-(2-methylphenyl)-*N*4-phenyl-6-(phenylmethyl)-2,4-pyrimidinediamine;
(e) *N*4-(2-methylphenyl)-6-(phenylmethyl)-2,4-pyrimidinediamine;
(f) *N*,*N*',*N*''-tris(4-methoxyphenyl)-2,4,6-pyrimidinetriamine;
(g) *N*,*N*'-bis(4-hexylphenyl)-6-(4-methoxyphenoxy)-2,4-pyrimidinediamine;
(h) *N*2,*N*4-bis(4-hexylphenyl)-*N*6,*N*6-dimethyl-2,4,6-pyrimidinetriamine;
(i) *N*,*N*',*N*''-tris(4-hexylphenyl)-2,4,6-pyrimidinetriamine;
(j) *N*2,*N*2-dimethyl-*N*4,*N*6-bis(4-methylphenyl)-2,4,6-pyrimidinetriamine;
(k) *N*,*N*',*N*''-tris(4-methylphenyl)-2,4,6-pyrimidinetriamine;
(l) *N*,*N*',*N*''-triphenyl-2,4,6-pyrimidinetriamine;
(m) *N*,*N*,*N*',*N*',*N*'',*N*''-hexakis(4-ethoxyphenyl)-2,4,6-pyrimidinetriamine;
(n) *N*4,*N*6-bis(2-chlorophenyl)-2,4,6-pyrimidinetriamine;
(o) *N*4,*N*6-bis(3-chlorophenyl)-2,4,6-pyrimidinethamine;
(p) *N*4,*N*6-bis(2-ethoxyphenyl)-2,4,6-pyrimidinetriamine;
(q) *N*4,*N*6-bis(4-ethoxyphenyl)-2,4,6-pyrimidinetriamine;
(r) *N*4,*N*6-bis(2-methylphenyl)-2,4,6-pyrimidinetriamine;
(s) *N*4,*N*6-bis(4-bromophenyl)-2,4,6-pyrimidinetriamine;
(t) *N*4,*N*6-bis(4-methylphenyl)-2,4,6-pyrimidinetriamine;
(u) *N*2,*N*4-bis(4-methoxyphenyl)-2,4,6-pyrimidinetriamine;
(v) *N*2,*N*4-bis(4-methylphenyl)-2,4,6-pyrimidinethamine;
(w) *N*,*N*',*N*''-tris(2,4,6-trinitrophenyl)-2,4,6-pyrimidinetriamine;
(x) *N*4,*N*6-bis(4-chlorophenyl)-2,4,6-pyrimidinetriamine;
(y) *N*4,*N*6-bis(4-methoxyphenyl)-2,4,6-pyrimidinetriamine;
(z) *N*2,*N*4,*N*6-trimethyl-*N*2,*N*4,*N*6-triphenyl pyrimidine-2,4,6-triyltriamine;
(aa) *N*4,*N*4-dimethyl-*N*2,*N*6-di-*p*-tolyl-pyrimidine-2,4,6-triyltriamine; and
(bb) *N*2,*N*4-diphenyl-pyrimidine-2,4,6-triyltriamine;
are not included;
a *N*-oxide, a pharmaceutically acceptable addition salt or a stereochemically isomeric form thereof.

**6.** A compound as defined in claim 1 wherein n is at least 1 and at least one $R^4$ is cyano.

**7.** A pharmaceutical composition comprising a pharmaceutically acceptable carrier and a therapeutically active amount of a compound as defined in any one of claims 5 or 6.

**8.** A process for preparing a pharmaceutical composition as defined in claim 7 characterized in that a therapeutically effective amount of a compound as defined in any one of claims 5 or 6 is intimately mixed with a pharmaceutically acceptable carrier.

**9.** A process for preparing a compound as defined in claim 5, characterized by reacting an intermediate of formula (II) wherein $W^1$ is a suitable leaving group with an amino derivative of formula (III) optionally in a solvent under a reaction-inert atmosphere, and optionally in the presence of an acid

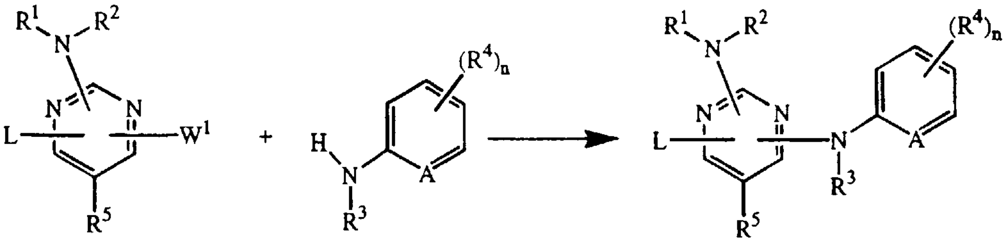

(II) (III) (I')

wherein $R^1$ to $R^5$, A, n and L are as defined in claim 1;
or if desired, converting compounds of formula (I') into each other following art-known transformations, and further, if desired, converting the compounds of formula (I'), into a therapeutically active non-toxic acid addition salt by treatment with an acid, or into a therapeutically active non-toxic base addition salt by treatment with a base, or conversely, converting the acid addition salt form into the free base by treatment with alkali, or converting the base addition salt into the free acid by treatment with acid; and, if desired, preparing stereochemically isomeric forms or *N*-oxides thereof..

**10.** The combination of a compound of formula (I) as defined in claim 1 and another antiretroviral compound.

**11.** A combination as claimed in claim 10 for use as a medicine.

**12.** A product containing (a) a compound of formula (I) as defined in claim 1, and (b) another antiretroviral compound, as a combined preparation for simultaneous, separate or sequential use in anti-HIV treatment.

**13.** A pharmaceutical composition comprising a pharmaceutically acceptable carrier and as active ingredients (a) a compound of formula (I) as defined in claim 1, and (b) another antiretroviral compound.

European Patent Office

# EUROPEAN SEARCH REPORT

Application Number
EP 98 20 1587

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| D,A<br><br><br>X | WO 91 18887 A (SMITH KLINE)<br>12 December 1991 (1991-12-12)<br>* claims 1-3; examples 1-38 *<br><br>----- | 1-4,8-13<br><br><br>5,8 | C07D239/50<br>C07D401/12<br>A61K31/505 |

TECHNICAL FIELDS SEARCHED (Int.Cl.6)
C07D
A61K

The present search report has been drawn up for all claims

| Place of search | Date of completion of the search | Examiner |
|---|---|---|
| THE HAGUE | 14 July 1999 | Francois, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT ON EUROPEAN PATENT APPLICATION NO.** EP 98 20 1587

This annex lists the patent family members relating to the patent documents cited in the above-mentioned European search report.
The members are as contained in the European Patent Office EDP file on
The European Patent Office is in no way liable for these particulars which are merely given for the purpose of information.

14-07-1999

| Patent document cited in search report | Publication date | Patent family member(s) | Publication date |
|---|---|---|---|
| WO 9118887 A | 12-12-1991 | AU 7971691 A | 31-12-1991 |

EPO FORM P0459

For more details about this annex : see Official Journal of the European Patent Office, No. 12/82